# EUROPÄISCHE PATENTSCHRIFT

(11) **EP 2 663 543 B1**
(45) Veröffentlichungstag und Bekanntmachung des Hinweises auf die Patenterteilung: **20.03.2019**
(21) Anmeldenummer: 12700258.2
(22) Anmeldetag: 04.01.2012
(51) Int. Cl.: B01J 31/02, B01J 31/06, B01J 31/24, C07C 51/00, C07C 51/083, C07C 53/02

(54) **CO2-HYDRIERUNGSVERFAHREN ZU AMEISENSÄURE**
CO2 HYDROGENATION METHOD FOR PRODUCING FORMIC ACID
PROCÉDÉ D'HYDROGÉNATION DE CO2 EN ACIDE FORMIQUE

(30) Priorität: 11.01.2011 DE 102011000077
(43) Veröffentlichungstag der Anmeldung: 20.11.2013
(73) Patentinhaber: Rheinisch-Westfälische Technische Hochschule Aachen, 52056 Aachen (DE)
(72) Erfinder: LEITNER, Walter, 52074 Aachen (DE); HINTERMAIR, Ulrich, 97228 Rottendorf (DE); WESSELBAUM, Sebastian, 52064 Aachen (DE)
(74) Vertreter: Michalski Hüttermann & Partner Patentanwälte mbB
(86) Internationale Anmeldenummer: PCT/EP2012/050111
(87) Internationale Veröffentlichungsnummer: WO 2012/095345

(56) Entgegenhaltungen:
- EP-A1- 0 181 078
- ZHAOFU ZHANG ET.AL.: "Hydrogenation of carbon dioxide is promoted by a task-specific ionic liquid", ANGEW. CHEM. INT. ED., Bd. 47, 2008, Seiten 1127-1129, XP002675924, in der Anmeldung erwähnt
- PHILLIP G. JESSOP ET.AL.: "Homogeneous catalytic hydrogenation of supercritical carbon dioxide", NATURE, Bd. 368, 7. Februar 1994 (1994-02-07), Seiten 231-233, XP002675925,

## Beschreibung

Die vorliegende Erfindung bezieht sich auf das Gebiet der Hydrierung von CO₂ zu Ameisensäure, insbesondere mit großer Reinheit und im großtechnischen Maßstab.

Ameisensäure ist ein großtechnisch vielfältiges eingesetztes Reagens der chemischen Industrie. Es ist essentiell für viele chemische Anwendungen und könnte - effiziente Darstellung vorausgesetzt - zukünftig auch als Wasserstoff- und/oder Kohlenmonoxid-Speicher im Bereich der Energien- und Chemiewirtschaft genutzt werden. Industriell wird Ameisensäure zur Zeit durch die katalytische Umsetzung von Kohlenmonoxid mit Natriumhydroxid oder Methanol sowie anschließender Hydrolyse gewonnen. Dieser Prozess ist jedoch insofern ungünstig, da die Aufreinigung der wäßrigen Ameisensäure problematisch ist, da Ameisensäure ein Azeotrop bildet und bei höheren Temperaturen auch die Neigung hat, sich wieder zu zersetzen.

Alternativ wurde die Darstellung von Ameisensäure durch Hydrierung von CO₂ vorgeschlagen. Diese Reaktion ist allerdings insofern problematisch, daß das ΔG für diese Reaktion positiv ist, d.h. rein aus den Edukten eine Darstellung thermodynamisch nicht möglich ist. Die Hydrierung von CO₂ ist möglich, wenn durch Zusatz von Stabilisatoren, meist von Basen eine Salz- bzw. Adduktbildung der entstehenden Ameisensäure erfolgt. Hierzu wurden mehrere Verfahren vorgeschlagen, wie z.B. in Zhang et. al, Angewandte Chemie, 2008, 47, 1127-1129, bei der ein Überschuß einer ionischen Flüssigkeit verwendet wird.

Weitere Hydrierungsverfahren sind in Jessop et al., Nature 368, 1994, S. 231-233 sowie der EP 181 078 bekannt.

Zur Zeit erfordern sämtliche Verfahren nach dem Stand der Technik den Einsatz (über)stöchiometrischer Mengen eines Stabilisators in Form einer Base. Dabei kommt es zur Bildung von Ameisensäuresalzen bzw. -Basenaddukten. Diese müssen in einem separaten Verfahrensschritt gespalten werden, um reine Ameisensäure freizusetzen. Somit stellen sich weitgehend dieselben Probleme bei der Abtrennung der Ameisensäure von der eingesetzten Base wie beim obigen Verfahren, bei dem Ameisensäure aus Kohlenmonoxid dargestellt wird. Zudem muss bei den meisten Verfahren gemäß des Stands der Technik aus toxikologischen und ökonomischen Gründen, sowie der Stabilität des Produkts der verwendete Katalysator abgetrennt werden. Diese Trenn- und Aufarbeitungsschritte machen die bisherigen Verfahren energetisch und wirtschaftlich unattraktiv.

Es stellt sich somit die Aufgabe, ein Hydrierungsverfahren von CO₂ zu Ameisensäure zu finden, welches in der Lage ist, die oben geschilderten Nachteile zumindest weitgehend zu überwinden, und durch welches eine effiziente kontinuierliche Reaktionsführung möglich ist.

Ein solches Verfahren wird durch Anspruch 1 der vorliegenden Erfindung bereitgestellt. Demgemäß wird ein Verfahren zur Hydrierung von CO₂ zu Ameisensäure vorgeschlagen, umfassend die Schritte:
a) katalytische Reaktion von CO₂ mit Wasserstoff in Gegenwart einer Base zu Ameisensäure
b) Zumindest teilweises Abführen der entstehenden Ameisensäure unter Einsatz von komprimiertem CO₂ aus dem Reaktionsbereich von Schritt a)
c) Freisetzen der Ameisensäure unter Entfernen des CO₂ aus Schritt b) Überraschenderweise hat sich herausgestellt, daß durch eine derartige Reaktionsführung die vorliegenden Nachteile bei den meisten Anwendungen derart überwunden werden können, daß ein großtechnischer Einsatz möglich wird.

Es hat sich herausgestellt, dass durch ein solches Verfahren bei den meisten Anwendungen innerhalb der vorliegenden Erfindung zumindest einer oder mehrere der folgenden Vorteile erzielt werden kann:
- Dadurch, daß unter Einsatz von komprimiertem CO₂ die Ameisensäure aus dem Reaktionsbereich entfernt wird, entfällt eine stoff- und energieintensive Trennung der Ameisensäure von den (zur Hydrierung notwendigen) Stabilisatoren. Weiterhin bleibt die Aktivität des Katalysators erhalten.
- Die Ameisensäure fällt in einer solchen Reinheit an, daß sie für die meisten technischen Prozesse ohne weitere Reinigungsschritte direkt verwendet werden kann
- Die bisherig notwendigen Abtrennungsschritte der Ameisensäure von Salz/Basen-Adduktverbindungen entfallen.
- Das Verfahren kann kontinuerlich durchgeführt werden, was einen effizienten großtechnischen Einsatz sehr erleichtert bzw. überhaupt erst möglich macht.

Der Term "komprimiertes CO₂" im Sinne der vorliegenden Erfindung bedeutet dabei insbesondere, daß CO₂ in gasförmiger (aber komprimierter), überkritischer oder flüssiger Form verwendet wird. Bevorzugt sind CO₂-Dichten im Bereich zwischen ≥0,2 g/mL und ≤1,2 g/mL, bevorzugt zwischen ≥0,3 g/mL und ≤0,9 g/mL. Je nach konkreter Ausgestaltung des Verfahrens kann das CO₂ auch (nur) flüssig und/oder überkritisch sein.

Der Term "unter Einsatz von komprimiertem CO₂" (Schritt b) soll nicht derart beschränkend verstanden werden, daß in Schritt b) ausschließlich komprimiertes CO₂ eingesetzt wird (auch wenn dies eine bevorzugte Ausführungsform der Erfindung ist. Je nach konkreter Ausgestaltung des Verfahrens können auch Flüssigkeitsgemische aus komprimiertem CO₂ mit weiteren Lösemitteln in Frage kommen, bevorzugt sind dabei Alkohole und Detergentien.

Der Term "Abführen" im Sinne der vorliegenden Erfindung bedeutet bzw. beinhaltet insbesondere, daß sich die Ameisensäure in CO₂ löst und somit extraktiv, über den Dampfdruck oder durch eine Kombination dieser Effekte aus dem Reaktionsbereich entfernt werden kann. Jedoch ist der Term "Abführen" im weitesten Sinne zu verstehen und nicht darauf beschränkt.

Bevorzugt wird das Verfahren kontinuierlich durchgeführt.

Der Term "kontinuierlich" im Sinne der vorliegenden Erfindung bedeutet bzw. beinhaltet insbesondere, dass der Katalysator einem Fluss von komprimiertem CO₂ (welches H₂ und ggf. Kosolventien beinhaltet) ausgesetzt ist, welcher zugleich als Transportvektor für die abgeführte Ameisensäure dient. Die Isolierung der Ameisensäure nach dem Reaktor wird bevorzugt durch Erniedrigung der CO₂-Dichte (Entspannen oder/und Erhitzen) oder durch Auswaschen aus dem CO2-Strom ebenfalls kontinuierlich durchgeführt.

Gemäß einer bevorzugten Ausführungsform der Erfindung liegt bei das CO₂ bei der Reaktion in Schritt a) im komprimierten Zustand vor. Dies erleichtert die Implementierung des Verfahrens, da so überwiegend in demselben Druckbereich gearbeitet werden kann. Bevorzugt liegt das CO₂ bei der Reaktion in Schritt a) überwiegend (d.h. zu ≥80%), noch bevorzugt ausschließlich (d.h. zu ≥99%) im flüssigen bzw. im überkritischen Zustand vor. Entsprechend wird gemäß einer bevorzugten Ausführungsform in Schritt c) entferntes CO₂ in Schritt a) wieder zugeführt.

Gemäß einer bevorzugten Ausführungsform der Erfindung umfasst die Base in Schritt a) eine ionische Flüssigkeit und/oder ionische Verbindung.

Unter dem Term "ionische Flüssigkeiten" werden insbesondere Verbindungen verstanden, die als Salze oder einer Salzmischung der Formel [A]ₙ⁺[Y]ⁿ⁻ mit n = 1 oder 2 entspricht, die unter Reaktionsbedingungen einen Schmelzpunkt aufweist, der kleiner als die Reaktionstemperatur ist, vorliegen.

Dies hat sich für das erfindungsgemäße Verfahren als günstig herausgestellt, da so bei den meisten Anwendungen einer oder mehrere der folgenden Vorteile erzielt werden kann:
- Durch den geringen Dampfdruck wird bei Schritt b) die Base nicht mit extrahiert sowie auch kein Solventverlust oder Kreuzkontamination stattfindet
- Das Gleichgewicht der Hydrierungsreaktion kann durch geeignete ionische Flüssigkeiten positiv in Richtung Produktbildung verschoben werden
- Die kontinuierliche Extraktion von gebildeter Ameisensäure macht katalytischen Einsatz des Stabilisators (basische IL) möglich, wobei weitere Aufreinigungsschritte vermieden werden
- Zudem zeigen viele Metallkatalysatoren insbesondere Übergangsmetallkatalysatoren ausgezeichnete Leistungsfähigkeit und Stabilität in ionischen Flüssigkeiten als Lösungsmittel.
- Es können erhebliche Mengen CO₂ in ionischen Flüssigkeiten gelöst werden (je nach Verbindung und konkreter Anwendung bis zu 70 mol%) ohne das eine allzu große Volumenexpansion der ionische Flüssigkeit selbst stattfindet und somit die physikalischen Eigenschaften dieser positive beeinflussen (geringer Viskosität, höhere Löslichkeit weiterer Gase wie z.B. Wasserstoff, gesteigerter Stofftransport, etc.).

Gemäß einer bevorzugten Ausführungsform der Erfindung ist die ionische Flüssigkeit bevorzugt ausgewählt aus der Gruppe der Onium-Ionen-Verbindungen, bevorzugt enthaltend Imidazoline, quart. Ammoniumsalze, quart. Phosphoniumsalze, alkylierte Pyridiniumsalze oder Mischungen daraus.

Noch bevorzugt sind dabei eine oder mehrere der folgenden Verbindungen:
- 4-Methylbutylipyridinium bis(trifluoromethylsulfonyl)imide ([MBP][NTf₂]) und 1-Ethyl-3-methyl-imidazolium bis(trifluoromethylsulfonyl)imide ([EMIM][NTf₂]) bzw. deren Analoga mit anderen geeigneten Anionen
- 1-(N,N-dethylaminoethyl)-2,3-dimethylimidazolium bis(trifluoromethylsulfonyl)imid ([EAMMIM][BTA]) bzw. Analoga mit anderen geeigneten Anionen
- 1-(N,N-dimethylaminoethyl)-2,3-dimethylimidazoliumsalze
- 1,3-di(N,N-dimethylaminoethyl)-2-methylimidazoliumsalze
- sowie 1-Alkyl-3-methylimidazoliumionen, Pyrrolidinium-, Pyridinium-, Tetraalkylammonium- oder Tetraalkylphosphoniumionen (organische Kationen) in Kombination mit Tetrafluorborat, Hexafluorphosphat, Halogenidanionen, bis(trifluormethansulfonyl)amid, Trifluormethansulfonat, Dicyanimid, Tosylat, Carboxylate (R-COO⁻ mit R = H, alkyl, aryl, etc; z.B. R = H Formiat, R = CH₃) Acetat oder n-Alkylsulfaten

Gemäß einer weiteren bevorzugten Ausführungsform der Erfindung umfasst die Base in Schritt a) ein hochmolekulares Amin. Dabei werden insbesondere Amine (bevorzugt Polyamine) mit einem Molekulargewicht von 1000 Da und mehr verstanden. Es hat sich herausgestellt, daß diese Amine eine so geringe Löslichkeit in komprimiertem CO₂ besitzen, daß diese ebenfalls für das erfindungsgemäße Verfahren in besonderer Weise geeignet sind. Bevorzugt sind polymere Amine oder Amin-Dendrimere. Beispiele für derartige Verbindungen sind polymergebundene (z.B. Polystyrol) Amine, Polyamine ausgehend von z. B. Benzylamin und Ethylendiamin oder anderen Monomeren, etc.) oder Dendrimere, Arborole und Kaskadenmoleküle, die Aminfunktionen enthalten (z.B. N((CH₂)ₙN((CH₂)ₙN((CH₂)ₙN((CH₂)ₙN(.....)))))₃ mit n ≥ 2).

Gemäß einer weiteren bevorzugten Ausführungsform der Erfindung ist die Base zumindest teilweise, bevorzugt überwiegend (d.h. zu ≥80 Gew-%), noch bevorzugt ausschließlich (d.h. zu ≥99 Gew-%) an einen festen Träger gebunden bzw. immobilisiert. Dies hat sich als besonders vorteilhaft herausgestellt, da so die Abführung der entstehenden Ameisensäure besonders einfach vonstatten gehen kann.

Der Term "Bindung" soll dabei im weitesten Sinne verstanden werden und sowohl kovalente Bindungen wie auch physikalische Bindungen (im Sinne von "Einschlüssen" etc.) oder Bindungen über elektrostatische, Wasserstoffbrücken- oder ionenartige Bindungen umfassen.

Bevorzugt poröse Träger bzw. Träger wie Silicate, Aluminate, Zeolithe, Aktivkohle, Graphite, MOFs, Graphitschäume (carbon aerogels) und/oder Carbon Nanotubes.

Gemäß einer bevorzugten Ausführungsform der Erfindung die Hydrierung in Schritt a) in Anwesenheit eines Übergangsmetallkatalysators erfolgt. Dies hat sich als besonders effektiv herausgestellt. Besonders bevorzugt sind Katalysatoren, die eins oder mehr der folgenden Übergangsmetalle: Ru, Co, Rh, Ir, Fe, Ni, Pd und Pt enthalten. Dabei sind wiederum heterogene Katalysatoren, nanopartikuläre Katalysatoren oder homogene geladene Katalysatoren besonders bevorzugt. Im besonderen bevorzugte Katalysatoren enthalten eine oder mehrere der folgenden Verbindungen:
- [Ru(Cl)₂(COD)]ₙ oder [Ru(cod)(methallyl)₂] mit PBu₄TPPMS (als Precursor-Materialien/Katalysatorvorstufe)
- Polystyrol-(CH₂)₃NH(CSCH₃)-{RuCl₃(PPh₃)}
- "Si"-(CH₂)₃NH(CSCH₃)-{RuCl₃(PPh₃)}
- [RhCl(PPh₃)₃], [{RhCl(cod)}₂], [RhCl(TPPTS)₃], [(dcpb)Rh(acac)], [RuCl₂{P(CH₃)₃}₄], [RuH₂{P(CH₃)₃}₄]und [RuCl₂(OAc)(PMe₃)₄] (Katalysatorvorstufe)
- [Cp*Ir(phen)Cl]Cl und [Ir^{III}PNP] (Katalysatorvorstufe)

Gemäß einer bevorzugten Ausführungsform der Erfindung weist der Katalysator in Schritt a) eine Löslichkeit in komprimierten CO₂ von ≤ 0,1g/L auf.

Gemäß einer bevorzugten Ausführungsform der Erfindung wird zumindest Schritt a) bei einem CO₂- Druck von ≥10 bar durchgeführt, bevorzugt ≥20 bar und am meisten bevorzugt ≥40 bar bis ≤500bar.

Gemäß einer bevorzugten Ausführungsform der Erfindung wird Schritt a) bei einem H₂-Druck von ≥5 bar durchgeführt, bevorzugt ≥10 bar und am meisten bevorzugt ≥20 bar bis ≤60bar.

Bevorzugt wird zumindest ein Teil - bevorzugt der überwiegende Teil, d.h. ≥80%, noch bevorzugt sämtliches, d.h. ≥99% - des in Schritt c) entfernten CO₂ Schritt a) und/oder b) wieder zugeführt.

Die vorliegende Erfindung bezieht sich ebenfalls auf eine Vorrichtung zur Durchführung des erfindungsgemäßen Verfahrens.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Zeichnungen, in denen - beispielhaft - mehrere Ausführungsbeispiele des erfindungsgemäßen Verfahrens dargestellt sind. In den Zeichnungen zeigt:
- Fig. 1: ein Fließbild einer Anlage zur Durchführung des erfindungsgemäßen Verfahrens gemäß einer ersten Ausführungsform der Erfindung (Beispiel I);
- Fig. 2: ein Diagramm, welches die "TON" (Turnover number, Im CO2-Strom nachweisbare Menge Ameisensäure relativ zum Metallgehalt des Katalysators (mol/mol)) bei der Durchführung des Verfahrens anhand Beispiel I illustriert;
- Fig. 3: ein Diagramm, welches den Verlauf der Gesamtumsatzzahl (TTON) und der Umsatzfrequenz (TOF) mit der Zeit bei der Durchführung des Verfahrens anhand Beispiel II illustriert;
- Fig. 4: ein NMR-Spektrum einer Probe während des Verfahrens anhand Beispiel II; sowie
- Fig. 5: ein Diagramm, welches den Verlauf der Gesamtumsatzzahl (TTON) und der Umsatzfrequenz (TOF) mit der Zeit bei der Durchführung des Verfahrens anhand Beispiel III illustriert.

### Beispiel I

Die vorliegende Erfindung wird anhand des Beispiels I untersucht, welches rein exemplarisch zu verstehen ist.

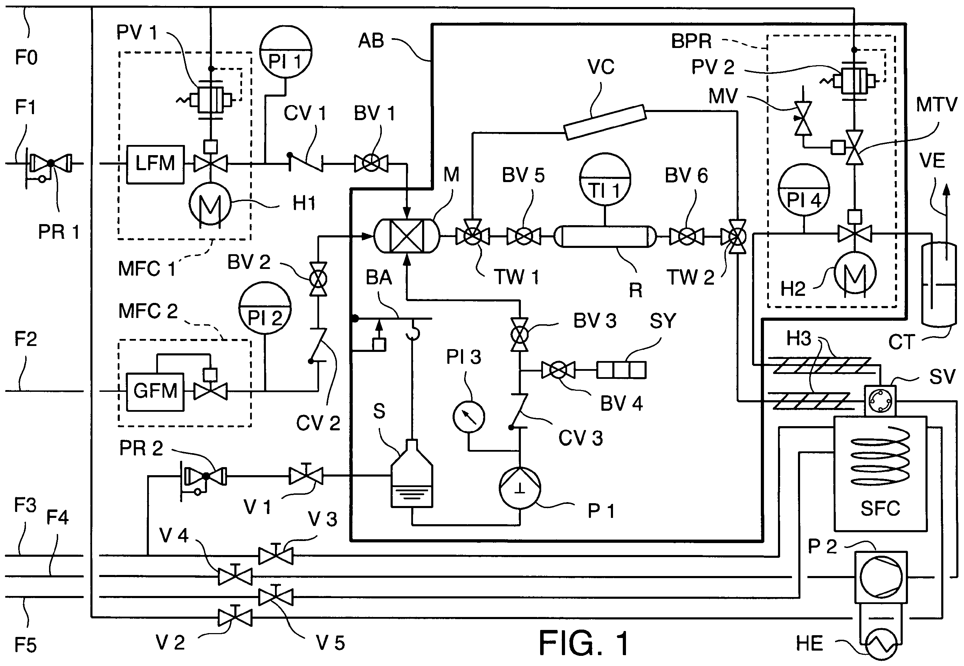

Fig. 1 ein Fließbild einer Anlage zur Durchführung des erfindungsgemäßen Verfahrens gemäß einer ersten Ausführungsform der Erfindung (Beispiel I). Dabei bedeuten:
F0 = Druckluft (6 bar), F1 = flüssig CO₂, F2 = H₂, F3 = N₂, F4 = flüssig CO₂ (ca. 40 - 70 bar), F5 = H₂, PR = Reduzierventil, MFC = Massenflussregler, LFM = Durchflussmengenmesser, GFM = Gasflussmengenmesser, H = Heizung, PI = Druckanzeige, PV = Proportionalventil, CV = Rückschlagventil, BV = Kugelhahn, AB = Inkubator, V = Feststellbares Ventil, M = Mischkammer, S = Reservoir für Ko-Solvens oder Derivatisierungsreagenz, BA = Waage, P = Kolbenpumpe, SY = Spülspritze, TW = Dreiwegehahn, R = Reaktor, VC = Hochdruck-Sichtzelle, TI = Temperaturanzeige, BPR = Rückdruck-Regelventil, MTV = Magnetschaltventil, MV = Feindosierventil, CT = Kühlfalle, VE = Entlüftung, SV = Hochdruck-Schaltventil, HE = Wärmetauscher, SFC = Supercritical Fluid Chromatograph).

Das erfindungsgemäße Verfahren findet (im wesentlichen) im Inkubator AB statt. Dabei wird zunächst in der Mischkammer M das CO₂ mit dem Wasserstoff gemischt (über geeignete Leitungen wie z.B: die F0 kann ggf. auch ein Cosolvens zugeführt werden). Anschließend erfolgt die Hydrierung im Reaktor R, über das Kugelventil BV6 verläßt dann die entstehende Ameisensäure (weitgehend) den Reaktionsbereich zusammen mit dem nicht reagierten CO₂. Zur Kontrolle der Reaktion ist ein Chromatograph "SFC" vorgesehen, der - bei großtechnischen Anwendungen oder anderen Ausführungsformen der Erfindung - auch entfallen kann. Im Druckkonstanthalter BPR erfolgt dann ggf. eine Abtrennung von CO₂ durch Entspannen der komprimierten mobilen Phase, welches über das Proportionalventil PV2 abgeführt und bei PV1 wieder zur weiteren Hydrierung rückgeführt werden kann. In der Kühlfalle CT zusammen mit der Entlüftung VE erfolgt dann die Abtrennung der (reinen) Ameisensäure vom CO₂. Je nach Anwendung kann auch hier das abgetrennte CO₂ wieder rückgeführt werden.

### Konkrete Beschreibung eines Versuchsdurchlaufs

### 1. Herstellung des immobilisierten Katalysators

Aminiertes Silicagel SiliaBond® Dethylamine (SiliCyle Inc, Partikelgröße 40-63 nm, mittlere Porengröße 600 nm, Oberfläche 500 m2/g, Porenvolumen 0.8 mL/g) wurde für 3 Stunden bei 80 °C und 0.1 mbar getrocknet und unter Argonatmosphäre gelagert. Für die genaue Dosierung des Metallkomplexes wurden eine Stammlösungen unter Argonatmosphäre angesetzt. Hierzu wurden 40 µmol [Ru(Cl₂)(COD)]ₙ eingewogen und in exakt 5 mL DCM gelöst (c = 8 µmol/mL). 0,34 mL (2,701 µmol) wurden entnommen und auf eine 3 Äquivalente PBu₄TPPMS gegeben. Die Mischung wurde am Hochvakuum zur Trockene eingedampft und in [EMIM][NTf₂] gelöst (c_{Ru} = 0,0027 mol/L). Nun wurde die in [EMIM][NTf₂] gelöste Vorstufe mit DCM versetztem Silicagel SiliaBond® Dethylamine vermengt und am Hochvakuum getrocknet, so dass die Beladung des Trägers 44,2 Gew.-% betrug. Anschließend erfolgte eine zweistündige Trocknung bei 0,1 mbar und 50 °C. Das geträgerte Katalysator-/Stabilisator-System wurde als Pulver erhalten.

### 2. Durchführung der Hydrierung

Unter Verwendung des festen Trägers aus 1. wurde eine Hydrierung in einem Versuchsaufbau gemäß des Fließbilds aus Fig. 1 durchgeführt. Dabei wurden wie folgt vorgegangen:

### Befüllen des Reaktors

Das Befüllen des Rohrreaktors wurde unter Argonatmosphäre durchgeführt. Hierzu wurde das Reaktorrohr an einem Ende geöffnet und mit 1 cm Glaswolle verschlossen. Darauf wurde die gewünschte Menge des geträgerte Katalysator-/Stabilisator-Systems (m = 4,257 g, V = 9,36 cm³, n_{Katalysator} = 2,701 µmol) gegeben und mit 1 cm Glaswolle fixiert und das Reaktorrohr verschlossen und in die Anlage eingebaut.

### Anfahren und Betrieb der Anlage

Zum Anfahren der Anlage blieben die Kugelhähne am Reaktor zunächst geschlossen und der Bypass wurde geöffnet. Der CO₂-Volumenstrom wurde auf 150 mL_{N}/min und der BPR auf 200 bar eingestellt. Wurde der gewünschte Druck erreicht wurde auf den Reaktor umgeschaltet um auch hier einen Druck von 200 bar voreinzustellen. War der Druck erreicht wurde wieder auf den Bypass umgeschaltet und der H₂-Volumenstrom zudosiert. War dieser Vordruck erreicht so wurden der CO₂-Volumenstrom auf 100 mL_{N}/min und der H₂-Volumenstrom auf 10 mL_{N}/min eingestellt. Als die Werte konstant blieben konnte vom Bypass auf den Rektor umgeschaltet werden. Dies war der Zeitpunkt zu dem die Reaktion gestartet wurde. Die Reaktionstemperatur betrug 50 °C. Nach 137 Stunden Betriebsdauer wurde die Reaktion beendet.

### Probenentnahme

Zum Sammeln des Produkts, das kontinuierlich mit Hilfe des CO2 extrahiert wurde, wurde der Extraktionsstrom nach dem BPR über eine Stahlkapillare in eine mit Reinstwasser und Glasperlen gefüllte Kühlfalle geleitet. Die Kühlfalle wurde periodisch ausgetauscht und der Inhalt bzgl. Ameisensäure analysiert.

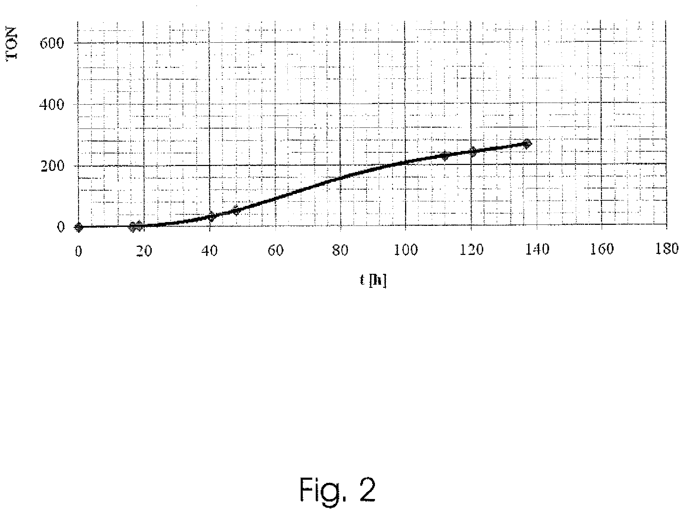

Fig. 2 zeigt den Verlauf der Umsatzzahl über die Zeit. Dabei konnten (bei noch völlig unoptimierten Bedingungen) Umsatzzahlen (TON) -Zahlen von maximal TON = 214 bei 137 Stunden im kontinuierlichen Betrieb erzielt werden.

### Beispiel II

Im hier beschriebenen Beispiel kommt die gleiche Reaktionsanlage wie in Beispiel I und Fig. 1 gezeigt zum Einsatz, wobei hier anstatt eines Strömungsrohrs ein kontinuierlicher Rührkessel Verwendung findet. Der Katalysator liegt hier homogen gelöst in einer IL-Phase vor.

### 1. Herstellung der Katalysatorlösung

Für die genaue Dosierung des Metallkomplexes wurden unter Argonatmosphäre 24 µmol [Ru(cod)(methallyl)₂] und 84 µmol PBu₄TPPMS (Verhältnis 1/3,5) in 3 mL DCM gelöst und von dieser Lösung 0,25 mL (2 µmol [Ru]) in ein weiteres Schlenk-Gefäß überführt. Als Additiv wurden 2 Äquivalente EMIMCl ebenfalls mittels Stammlösung in DCM zugegeben. 1 mL der IL 1-(N,N-dethylaminoethyl)-2,3-dimethylimidazolium bis(trifluoromethylsulfonyl)imid ([EAMMIM][BTA]) wurde zugegeben und das Gemisch zur Entfernung des DCM für 2 h bei 70 °C im HV gerührt.

### 2. Durchführung der Hydrierung

Unter Verwendung der in 1. hergestellten Katalysator/Stabilisator-Lösung wurde eine Hydrierung in einem Versuchsaufbau gemäß dem Fließbild aus Fig. 1 durchgeführt. Dabei wurde wie folgt vorgegangen:

### Befüllen des Reaktors

Die Katalysator/Stabilisator-Lösung wurde unter Argonatmosphäre in den 10 mL Rührkessel überführt und dessen Kugelhähne verschlossen. Der so präparierte Reaktor wurde in die Anlage eingebaut.

### Anfahren und Betrieb der Anlage

Das Anfahren und der Betrieb der Anlage erfolgte wie in Beispiel I beschrieben, mit der Abweichung: Es wurde im Bypass-Betrieb ein konstanter CO₂-Volumenstrom von 200 mL_{N}/min und ein H₂-Volumenstrom von 20 mL_{N}/min bei 200 bar eingestellt. Es wurde von Bypass auf Reaktor umgestellt und der Reaktor so 30 min gespült, um die Atmosphäre im Reaktor derer während des kontinuierlichen Betriebs gleich zu machen. Anschließend wurde für 20 h erneut auf Bypass umgestellt, um im Reaktor die Gleichgewichtskonzentration an Ameisensäure entstehen zu lassen, was die Extraktion von Beginn an verbessert. Nach 20 h wurden der CO₂ und H₂-Strom wieder durch den Reaktor geleitet. Dieser Zeitpunkt wird im Folgenden als Startpunkt der kontinuierlichen Reaktion betrachtet. Die Reaktion wurde nach 211 Stunden Betriebsdauer gestoppt.

### Probenentnahme

Die Probenentnahme erfolgte wie in Beispiel I beschrieben.

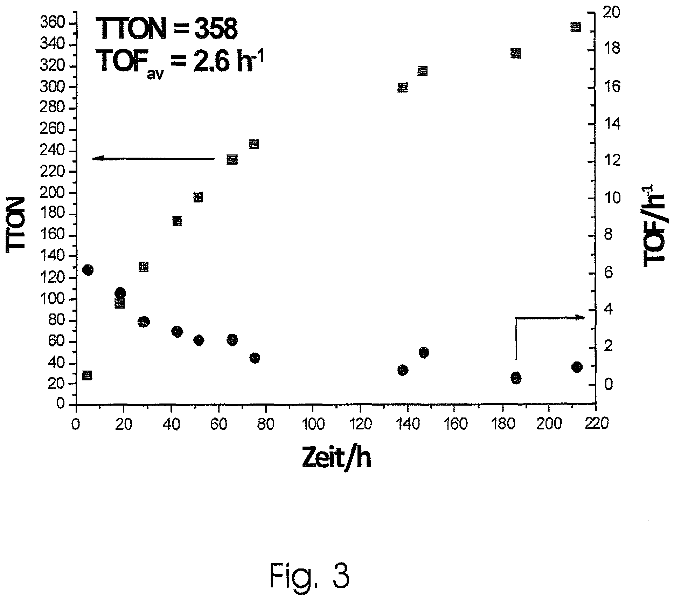

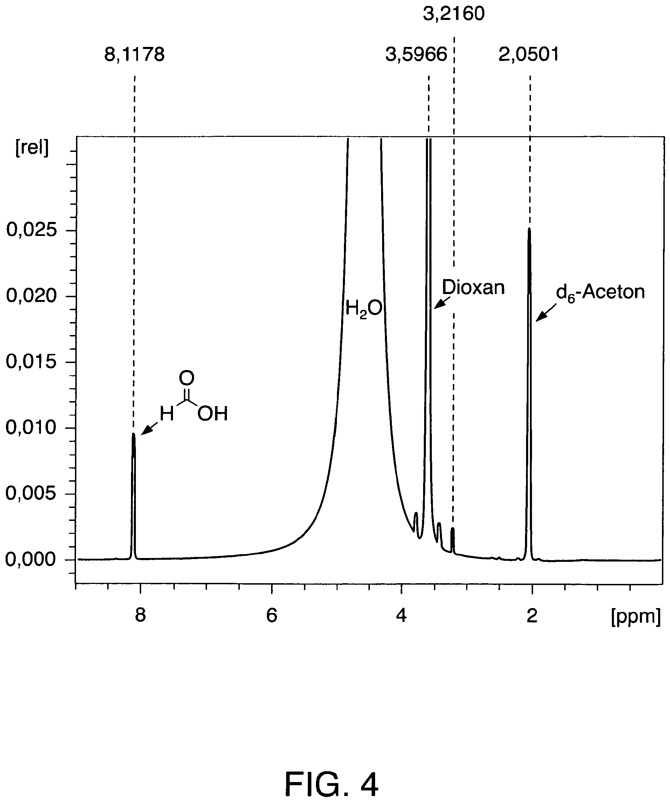

Fig. 3 zeigt den Verlauf der Gesamtumsatzzahl (TTON) und der Umsatzfrequenz (TOF) mit der Zeit. Dabei konnte eine TTON von 358 nach 211 Stunden im kontinuierlichen Betrieb erreicht werden. Fig. 4 zeigt beispielhaft die ¹H-NMR-Analyse des Kühlfalleninhalts mit Dioxan als internem Standard. Bei dem Signal bei 3.2 ppm handelt es sich um Spuren Methanol, mit welchem die Anlage zuvor gereinigt wurde.

### Beispiel III

Das hier beschriebene Beispiel ist wie das Beispiel II, nur mit Einsatz eines heterogenen, polymergebundenen Amins QuadraPure™-DMA anstelle einer Amino-funktionalisierten IL.

### 1. Herstellung der Katalysatorlösung

Für die genaue Dosierung des Metallkomplexes wurden unter Argonatmosphäre 24 µmol [Ru(cod)(methallyl)₂] und 84 µmol PBu₄TPPMS (Verhältnis 1/3,5) in 3 mL DCM gelöst und von dieser Lösung 0,25 mL (2 µmol [Ru]) in ein weiteres Schlenk-Gefäß überführt. Als Additiv wurden 2 Äquivalente EMIMCl ebenfalls mittels Stammlösung in DCM zugegeben. 1 mL der IL [EMIM][BTA] wurde zugegeben und das Gemisch zur Entfernung des DCM für 2 h bei 70 °C im HV gerührt.

### 2. Durchführung der Hydrierung

Unter Verwendung der in 1. hergestellten Katalysator/Stabilisator-Lösung wurde eine Hydrierung in einem Versuchsaufbau gemäß dem Fließbild aus Fig. 1 durchgeführt. Dabei wurde wie folgt vorgegangen:

### Befüllen des Reaktors

0,5 g QuadraPure™-DMA sowie die Katalysator/Stabilisator-Lösung wurden unter Argonatmosphäre in den 10 mL Rührkessel gegeben und dessen Kugelhähne verschlossen. Der so präparierte Reaktor wurde in die Anlage eingebaut.

### Anfahren und Betrieb der Anlage

Das Anfahren und der Betrieb der Anlage erfolgte wie in Beispiel II beschrieben, mit den Abweichungen: Es wurde im Bypass-Betrieb ein konstanter CO₂- Volumenstrom von 200 mL_{N}/min und ein H₂-Volumenstrom von 20 mL_{N}/min bei 200 bar eingestellt. Es wurde von Bypass auf Reaktor umgestellt um die kontinuierliche Reaktion zu starten. Die Reaktion wurde nach 190 Stunden Betriebsdauer gestoppt.

### Probenentnahme

Die Probenentnahme erfolgte wie in Beispiel I beschrieben.

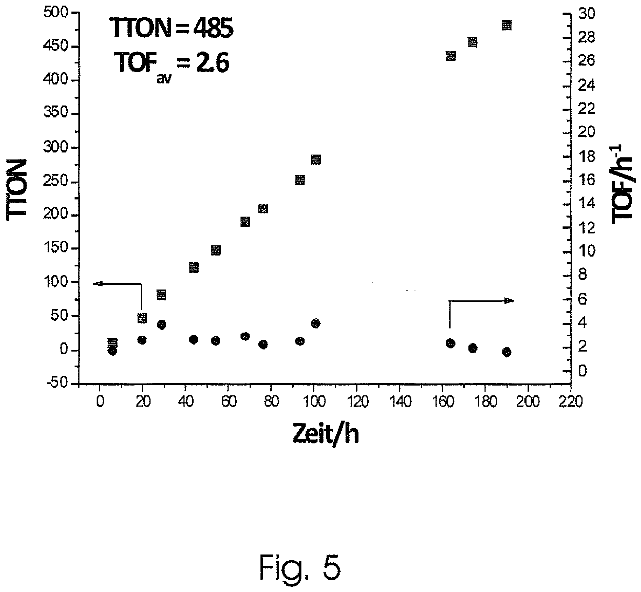

Fig. 5 zeigt den Verlauf der Gesamtumsatzzahl (TTON) und der Umsatzfrequenz (TOF) mit der Zeit. Dabei konnte eine TTON von 485 nach 190 Stunden im kontinuierlichen Betrieb erreicht werden.

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Der Fachmann erkennt, dass Variationen, Modifikationen und andere Ausführungen, die hier beschrieben werden, ebenfalls auftreten können ohne von dem Erfindungsgedanken und dem Umfang der Erfindung abzuweichen. Entsprechend ist die obengenannte Beschreibung beispielhaft und nicht als beschränkend anzusehen. Das in den Ansprüchen verwendetet Wort umfassen schließt nicht andere Bestandteile oder Schritte aus. Der unbstimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werde kann. Der Umfang der Erfindung ist in den folgenden Ansprüchen definiert und den dazugehörigen Äquivalenten.

## Patentansprüche

1. Verfahren zur Hydrierung von CO₂ zu Ameisensäure, umfassend die Schritte
a) katalytische Reaktion von CO₂ mit Wasserstoff in Gegenwart einer Base zu Ameisensäure
b) Zumindest teilweises Abführen der entstehenden Ameisensäure unter Einsatz von komprimiertem CO₂ aus dem Reaktionsbereich von Schritt a) während der katalytischen Reaktion von CO₂ mit Wasserstoff
c) Freisetzen der Ameisensäure unter Entfernen des CO₂ aus Schritt b)

2. Verfahren nach Anspruch 1, wobei das Verfahren kontinuierlich durchgeführt wird.

3. Verfahren nach Anspruch 1 oder 2, wobei das CO₂ bei der Reaktion in Schritt a) im komprimierten Zustand vorliegt.

4. Verfahren nach einem der Ansprüche 1 bis 3, wobei die Base in Schritt a) eine ionische Flüssigkeit umfasst

5. Verfahren nach einem der Ansprüche 1 bis 4, wobei die ionische Flüssigkeit bevorzugt ausgewählt ist aus der Gruppe enthaltend Imidazoline, quart. Ammoniumsalze, quart. Phosphoniumsalze oder Mischungen daraus

6. Verfahren nach einem der Ansprüche 1 bis 5, wobei die Base in Schritt a) ein hochmolekulares Amin mit einem Molekulargewicht von 1000 Da und mehr ist.

7. Verfahren nach einem der Ansprüche 1 bis 6, wobei die Base an einen festen Träger gebunden ist.

8. Verfahren nach einem der Ansprüche 1 bis 7, wobei die Hydrierung in Schritt a) in Anwesenheit eines Übergangsmetallkatalysators erfolgt.

9. Verfahren nach einem der Ansprüche 1 bis 8, wobei zumindest Schritt a) bei einem CO₂- Druck von ≥10 bar durchgeführt wird

10. Verfahren nach einem der Ansprüche 1 bis 9, wobei zumindest ein Teil des in Schritt c) entfernten CO₂ Schritt a) und/oder b) wieder zugeführt wird.

## Claims

1. Method for hydrogenation of CO₂ into formic acid comprising the steps:
a) catalytic reaction of CO₂ with hydrogen in the presence of a base into formic acid;
b) at least partially discharging the resulting formic acid by use of compressed CO₂ from the reaction zone of step a) during the catalytic reaction of CO₂ with hydrogen; and
c) releasing the formic acid with removal of the CO₂ from step b).

2. Method according to claim 1, wherein the method is carried out continuously.

3. Method according to claim 1 or 2, wherein the CO₂ is present in the compressed state in the reaction in step a).

4. Method according to any one of claims 1 to 3, wherein the base in step a) comprises an ionic liquid.

5. Method according to any one of claims 1 to 4, wherein the ionic liquid is preferably selected from the group comprising imidazolines, quaternary ammonium salts, quaternary phosphonium salts or mixtures thereof.

6. Method according to any one of claims 1 to 5, wherein the base in step a) is a high molecular weight amine having a molecular weight of 1000 Da and more.

7. Method according to any one of claims 1 to 6, wherein the base is bound to a solid carrier.

8. Method according to any one of claims 1 to 7, wherein the hydrogenation in step a) is carried out in the presence of a transition metal catalyst.

9. Method according to any one of claims 1 to 8, wherein at least step a) is carried out at a CO₂ pressure of ≥ 10 bar.

10. Method according to any one of claims 1 to 9, wherein at least a part of the CO₂ removed in step c) is returned to step a) and/or step b).

## Revendications

1. Procédé pour l'hydrogénation de CO₂ en acide formique, comprenant les étapes de
a) réaction catalytique de CO₂ avec de l'hydrogène, en présence d'une base, pour former de l'acide formique
b) élimination au moins partielle de l'acide formique résultant à l'aide de CO₂ comprimé de la zone de réaction de l'étape a) pendant la réaction catalytique de CO₂ avec de l'hydrogène
c) libération de l'acide formique par élimination du CO₂ de l'étape b)

2. Procédé selon la revendication 1, dans lequel le procédé est mis en oeuvre de façon continue.

3. Procédé selon la revendication 1 ou la revendication 2, dans lequel le CO₂ dans la réaction de l'étape a) est à l'état comprimé.

4. Procédé selon l'une quelconque des revendications 1 à 3, dans lequel la base dans l'étape a) comprend un liquide ionique

5. Procédé selon l'une des revendications 1 à 4, dans lequel le liquide ionique est de préférence choisi dans le groupe comprenant les imidazolines, sels d'ammonium quat., sels de phosphonium quat. ou des mélanges de ceux-ci

6. Procédé selon l'une quelconque des revendications 1 à 5, dans lequel la base dans l'étape a) est une amine à haut poids moléculaire ayant un poids moléculaire de 1000 Da ou plus.

7. Procédé selon l'une quelconque des revendications 1 à 6, dans lequel la base est liée à un support solide.

8. Procédé selon l'une quelconque des revendications 1 à 7, dans lequel l'hydrogénation dans l'étape a) a lieu en présence d'un catalyseur à base de métal de transition.

9. Procédé selon l'une quelconque des revendications 1 à 8, dans lequel au moins l'étape a) est réalisée à une pression de CO₂ ≥ 10 bar.

10. Procédé selon l'une quelconque des revendications 1 à 9, dans lequel au moins une partie de CO₂ éliminé dans l'étape c) est renvoyée à l'étape a) et/ou b).
